# EUROPÄISCHE PATENTSCHRIFT

(11) **EP 1 519 301 B1**
(45) Veröffentlichungstag und Bekanntmachung des Hinweises auf die Patenterteilung: **08.09.2010**
(21) Anmeldenummer: 04090334.6
(22) Anmeldetag: 27.08.2004
(51) Int. Cl.: G06K 9/00

(54) **Vorrichtung zur klassifikation physiologischer Ereignisse**
Apparatus for classifying physiological events
Dispositif de classification d'événements physiologiques

(30) Priorität: 29.09.2003 US 674270
(43) Veröffentlichungstag der Anmeldung: 30.03.2005
(73) Patentinhaber: Biotronik GmbH & Co. KG, 12359 Berlin (DE)
(72) Erfinder: De Voir, Christopher S., Tigard, Oregon 97223-5602 (US); Schomburg, Richard A., Hillsboro, oregon 97123-9048 (US)
(74) Vertreter: Lindner-Vogt, Karin L.

(56) Entgegenhaltungen:
- US-A- 5 638 823
- US-A- 6 135 966
- F. WANG, R. QUINIOU, G. CARRAULT, AND M.-O. CORDIER: "Learning Structural Knowledge from the ECG" ISMDA 2001, LNCS 2199, 2001, Seiten 288-294, XP002398786 Springer-Verlag Berlin Heidelberg
- WIGGINS, M; ZHAO, L; VACHTSEVANOS, G; LITT, B: "Non-invasive, cardiac risk stratification using wavelet coefficients" WSEAS TRANSACTIONS ON COMPUTERS, Bd. 2, Nr. 3, Juli 2003 (2003-07), Seiten 720-723, XP002398787
- AL-FAHOUM ET AL: "COMBINED WAVELET TRANSFORMATION AND RADIAL BASIS NEURAL NETWORKS FOR CLASSIFYING LIFE-THREATENING CARDIAC ARRHYTHMIAS" MEDICAL AND BIOLOGICAL ENGINEERING AND COMPUTING, SPRINGER, HEILDELBERG, DE, Bd. 37, Nr. 5, September 1999 (1999-09), Seiten 566-573, XP000852460 ISSN: 0140-0118
- XUESONG YE ET AL: "Noninvasive detection of coronary artery disease based on heart sounds" ENGINEERING IN MEDICINE AND BIOLOGY SOCIETY, 1998. PROCEEDINGS OF THE 20TH ANNUAL INTERNATIONAL CONFERENCE OF THE IEEE HONG KONG, CHINA 29 OCT.-1 NOV. 1998, PISCATAWAY, NJ, USA,IEEE, US, Bd. 3, 29. Oktober 1998 (1998-10-29), Seiten 1546-1548, XP010320347 ISBN: 0-7803-5164-9
- LEUNG T S ET AL: "Classification of heart sounds using time frequency method and artificial neural networks" ENGINEERING IN MEDICINE AND BIOLOGY SOCIETY, 2000. PROCEEDINGS OF THE 22ND ANNUAL INTERNATIONAL CONFERENCE OF THE IEEE 23-28 JULY 2000, PISCATAWAY, NJ, USA,IEEE, Bd. 2, 23. Juli 2000 (2000-07-23), Seiten 988-991, XP010529082 ISBN: 0-7803-6465-1
- DUDA R O ET AL: "Pattern Classification - PNNs and RBFs" PATTERN CLASSIFICATION, NEW YORK, JOHN WILEY & SONS, US, 2001, Seite 172-173,324-325, XP002398788 ISBN: 0-471-05669-3

## Beschreibung

Die vorliegende Erfindung bezieht sich auf eine Vorrichtung zur Klassifikation physiologischer Ereignisse

Physiologische Ereignisse rufen physiologische Signale hervor oder stellen selbst Signale dar, anhand derer sie klassifiziert werden können. Das Klassifizieren von physiologischen Ereignissen bzw. Signalen ist insbesondere bei implantierbaren medizinischen Geräten, wie etwa Herzschrittmachern oder implantierbaren Defibrillatoren nützlich, um behandlungsbedürftige Ereignisse von solchen, die nicht behandlungsbedürftig sind, oder Ereignisse, für die unterschiedliche Behandlungen angezeigt sind, voneinander zu unterscheiden. Anhand der Klassifikation wird das implantierbare medizinische Gerät in die Lage versetzt, selbständig die ggf. notwendige Behandlung auszulösen.

Aus Wang et al. "Learning Structure Knowledge from the ECG", ISMDA 2001, LNCS 2199, Seiten 288 bis 294, Axel Springer Verlag Berlin-Heidelberg 2001 ist es bekannt, ein Elektrokardiogramm einer Wavelet-Transformation zu unterziehen und die Wavelet-Transformierten einem probabilistischen neuronalen Netzwerk zuzuführen.

Auch aus AI-Fahoum und I. Howitt "Combined Wavelet Transformation and Radial Basis Neural Networks for Classifying Life-Threatening Cardiac Arrhythmias" in Medical and Biological Engineering and Computing 1999, Bd. 37, S. 566 bis 573, ist die Analyse von Elektrokardiogrammen mit Hilfe der Wavelet-Transformation und eines neuronalen Netzwerkes bekannt.

Leung T. S. et al. beschreiben in "Classification of Heart Sounds Using Time Frequency Method and Artificial Neural Networks" in proceedings of the 22nd Annual EMBS International Conference, July 23 - 28, 2000, Chicago, Illinois, USA, die Analyse von Phonokardiogrammen mit Hilfe künstlicher neuronaler Netzwerke.

Auch in US 6,135,966 ist die Analyse eines physiologischen Signals, nämlich des arteriellen Druckverlaufs, mit Hilfe der Wavelet-Transformation und eines neuronalen Netzwerkes bekannt.

In "Pattern Classification" sind neuronale Netze im Allgemeinen beschrieben und in Kapitel 4.3.5 probabilistische neuronale Netze im Speziellen. Kapitel 6.10 beschreibt darüber hinaus das Training eines neuronalen Netzes mit zeitverschobenen Eingangssignalen.

Bisherige Vorrichtungen zur Klassifikation von physiologischen Ereignissen, insbesondere von intrakardialen Ereignissen, in implantierbaren medizinischen Geräten beruhen im Wesentlichen auf einem Filtern der Signalform sowie auf dem Vorsehen eines Schwellenwertes oder mehrerer Schwellenwerte in Kombination mit einer Zeitanalyse des Über-/Unterschreitens des Schwellenwertes bzw. der Schwellenwerte.

Um mit den bisherigen Vorrichtungen eine akzeptable Empfindlichkeit auf die Signale von physiologischen Ereignissen sowie eine akzeptable Unterscheidbarkeit von Ereignissen zu erzielen, ist es notwendig, während des Herzzyklus, in den ein Ereignis fällt, das Aufnehmen von weiteren physiologischen Signalen auszusetzen. Dieses Aussetzen schließt jedoch das zuverlässige Erkennen verschiedener wichtiger Ereignisklassen von intrakardialen Ereignissen sowie deren wirksame Behandlung aus, so bspw. eine anormale Beziehung zwischen den beiden Herzkammern.

Es ist daher Aufgabe der vorliegenden Erfindung, eine verbesserte Vorrichtung zur Klassifikation von physiologischen Ereignissen, insbesondere intrakardialer Ereignisse, zur Verfügung zu stellen, welche die genannten Nachteile zu überwinden hilft.

Diese Aufgabe wird durch eine Vorrichtung zur Klassifikation physiologischer Ereignisse nach Anspruch 1 gelöst. Die abhängigen Ansprüche enthalten weitere Ausgestaltungen der Erfindung.

Eine erfindungsgemäße Vorrichtung zum Klassifizieren physiologischer Ereignisse weist einen Signaleingang zum Eingeben eines auf einem physiologischen Ereignis beruhenden, oder das es darstellenden oder repräsentierenden physiologischen Signals auf sowie eine das physiologische Signal anhand seiner Signalform klassifizierende Klassifikationseinheit. In der erfindungsgemäßen Vorrichtung umfasst die Klassifikationseinheit:
- eine Transformationseinheit, die derart zum Durchführen einer Transformation des physiologischen Signals ausgestaltet ist, dass sie als Ausgangssignal eine Anzahl das physiologische Signalrepräsentierender und auf der Transformation beruhender Werte ausgibt; und
ein mit der Transformationseinheit zum Empfang der Werte verbundenes probabilistisches neuronales Netzwerk, das eine Anzahl physiologischer Ereignisse repräsentierender Ereignisklassen enthält (die typischerweise in Form von sog. Knoten implementiert sind), die wiederum jeweils durch einen Satz von Vergleichswerten repräsentiert sind, wobei probabilistische neuronale Netzwerke dazu ausgestaltet ist, anhand des Vergleichs der Werte mit den Vergleichswerten eine Zuordnung des von den Werten repräsentierten physiologischen Signals zu einer der Ereignisklassen vorzunehmen.

Das Klassifizieren erfolgt also durch das Zuordnen des das physiologische Ereignis darstellenden oder repräsentierenden physiologischen Signals zu einer Ereignisklasse. Als physiologisches Signal soll hierbei auch ein vor dem Eingeben in die Klassifikationseinheit aufbereitetes, bspw. normiertes, gefiltertes, justiertes oder ähnlich umgeformtes physiologisches Signal angesehen werden.

Die Kombination aus Transformationseinheit und probabilistischem neuronalen Netzwerk bildet eine wirksame Klassifikationseinheit zur Klassifikation von Signalen intrakardialer Ereignisse oder anderer Ereignisse oder anderen Signalen biologischen Ursprungs.

Die Transformationseinheit ist eine auf einer Wavelet-Transformation beruhende Transformationseinheit, d.h. sie ist zum Durchführen der Transformation anhand von Wavelets und einer die auszugebenden Werte unter Verwendung der Wavelets bestimmenden Transformationsregel ausgestaltet.

Die Wavelet-Transformation ist einfach durchzuführen und ermöglicht es, Signale mit relativ wenigen Werten (Koeffizienten) zu repräsentieren. Gleichzeitig beleibt bei der Wavelet-Transformation genügend Information über das Signal erhalten, um eine zuverlässige Klassifikation probabilistischen neuronalen Netzwerk zu gewährleisten. Zudem bietet die Wavelet-Transformation die Möglichkeit, die Transformation innerhalb der für das Berechnen der Transformation bestehenden mathematischen Grenzen an das effektive Erkennen einzelner Ereignisklassen anzupassen.

Die mittels der Wavelet-Transformation gewonnenen Werte enthalten vorzugsweise Werte, die ein Stammwavelet beschreiben und zusätzlich Skalierungswerte und Translationswerte (Koeffizienten), die bezogen auf ein jeweiliges Stammwavelet die Form des Eingangssignals (physiologisches Signal) charakterisieren.

Die Vergleichbarkeit der Werte und der Vergleichswerte im probabilistischen neuronalen Netzwerk ist in besonderem Maße gewährleistet, wenn die Vergleichswerte auf einer Transformation beruhen, bei der die gleichen Wavelets und die gleiche Transformationsregel wie in der Transformationseinheit Verwendung finden.

In einer Ausgestaltung des probabilistischen neuronalen Netzwerks umfasst dieses zum Durchführen der Zuordnung des von den Werten repräsentierten physiologischen Signals zu einer der Ereignisklassen:
- mindestens eine Ermittlungseinheit zum Bestimmen von Zugehörigkeitswahrscheinlichkeiten des physiologischen Signals zu den Ereignisklassen auf der Basis des Vergleichs der Werte mit den Vergleichswerten der jeweiligen Ereignisklasse und zum Ausgeben der ermittelten Zugehörigkeitswahrscheinlichkeiten; und
- eine mit der Ermittlungseinheit zum Empfang der Zugehörigkeitswahrscheinlichkeiten verbundene Selektionseinheit, die zum Extrahieren der höchsten Zugehörigkeitswahrscheinlichkeit aus den Zugehörigkeitswahrscheinlichkeiten sowie zum Zuordnen des physiologischen Signals zu der Ereignisklasse mit der höchsten Zugehörigkeitswahrscheinlichkeit ausgebildet ist.

In der erfindungsgemäßen Vorrichtung sind für mindestens eine Ereignisklasse zwei oder mehr Sätze von dieselbe Ereignisklasse repräsentierenden Vergleichswerten bzw. -koeffizienten vorhanden. Für solche Ereignisklassen, ist die Ermittlungseinheit insbesondere zum Bestimmen mehrerer Zugehörigkeitswahrscheinlichkeiten für jede dieser Ereignisklassen ausgebildet. Die Selektionseinheit ist dann derart ausgebildet, dass sie für diejenigen Ereignisklassen, die zwei oder mehr Sätze von dieselbe Ereignisklasse repräsentierenden Vergleichswerten aufweisen, Mittelwerte der entsprechenden Zugehörigkeitswahrscheinlichkeiten bildet und beim Extrahieren der höchsten Zugehörigkeitswahrscheinlichkeit die Mittelwerte statt der Einzelwerte heranzieht. Mit dieser Weiterbildung kann die Empfindlichkeit der Vorrichtung auf Signalrauschen reduziert werden. Das Signalrauschen kann insbesondere dann zu Klassifizierungsfehlern und infolge dessen zu einer geringeren Klassifikationsgenauigkeit führen, wenn eine gewisse Variationsbreite in dasselbe physiologische Ereignis repräsentierenden physiologischen Signalen vorhanden ist. Mit der erwähnten Weiterbildung der erfindungsgemäßen Vorrichtung lassen sich derartige Variationen durch verschiedne Sätze von Vergleichswerten berücksichtigen, wodurch die mit einem unverrauschten Signal mögliche Klassifikationsgenauigkeit auch bei solchen Signalen weitgehend erhalten bleibt, deren Rauschpegel ohne Berücksichtigung der Variationen die Klassifikationsgenauigkeit deutlich reduzieren würde.

In der erfindungsgemäßen Vorrichtung ist der Transformationseinheit eine Justiereinheit zum Zentrieren des physiologischen Signals in einem Zeitfenster vorgegebener Fensterweite und zum Ausgeben des zentrierten physiologischen Signals an die Transformationseinheit vorgeschaltet, um ein einheitliches Eingabeformat für die auftretenden physiologischen Signale zu erzielen. Die oben erwähnten Variationen in an sich gleichen physiologischen Signalen können dabei bspw. in Form von Offsets in der von der Justiereinheit vorgenommenen Zentrierung auftreten. Diese Offsets würden, wenn sie unberücksichtigt blieben, die Empfindlichkeit der Vorrichtung gegenüber Signalrauschen erhöhen und dadurch die Klassifika tionsgenauigkeit reduzieren. Um die Offsets berücksichtigen zu können, entsprechen bei denjenigen Ereignisklassen, die zwei oder mehr Sätze von dieselbe Ereignisklasse repräsentierenden Vergleichswerten umfassen, die Sätze von Vergleichswerten unterschiedlichen Offsets in der Zentrierung des zentrierten physiologischen Signals.

Die erfindungsgemäße Vorrichtung, die sowohl in Form von Hardware als auch in Form von Software implementiert werden kann, eignet sich insbesondere zum Einsatz in einem implantierbaren medizinischen Gerät, wie etwa in einem Herzschrittmacher oder Defibrillator.

Weitere Merkmale, Eigenschaften und Vorteile der erfindungsgemäßen Vorrichtung ergeben sich aus der nachfolgenden Beschreibung eines Ausführungsbeispiels unter Bezugnahme auf die beiliegenden Zeichnungen.
- Fig. 1: zeigt ein Ausführungsbeispiel für die erfindungsgemäße Vorrichtung zum Klassifizieren physiologischer Ereignisse.
- Fig. 2: zeigt ein Paar von Testsignalen, anhand derer die Arbeitsweise der erfindungsgemäßen Vorrichtung erläutert wird.
- Fig. 3: zeigt die mittels Wavelet-Transformation gewonnenen Koeffizienten der beiden in Fig. 2 dargestellten Testsignale.
- Fig. 4: zeigt den Effekt, den ein Offset in der Zentrierung der beiden Testsignale in einem Zeitfenster auf das Klassifizierungsergebnis hat.
- Fig. 5: zeigt den Effekt, den ein Offset in der Zentrierung der beiden Testsignale in einem Zeitfenster auf das Klassifizierungsergebnis hat, wenn Offsets durch zwei oder mehr Sätze von dieselbe Ereignisklasse repräsentierenden Vergleichskoeffizienten Berück- sichtigung finden.
- Fig. 6: zeigt die Fehlerrate beim Klassifizieren für verschiedene beim Zentrieren im Zeitfenster auftretende Offsets in Abhängigkeit vom Signalrauschen.

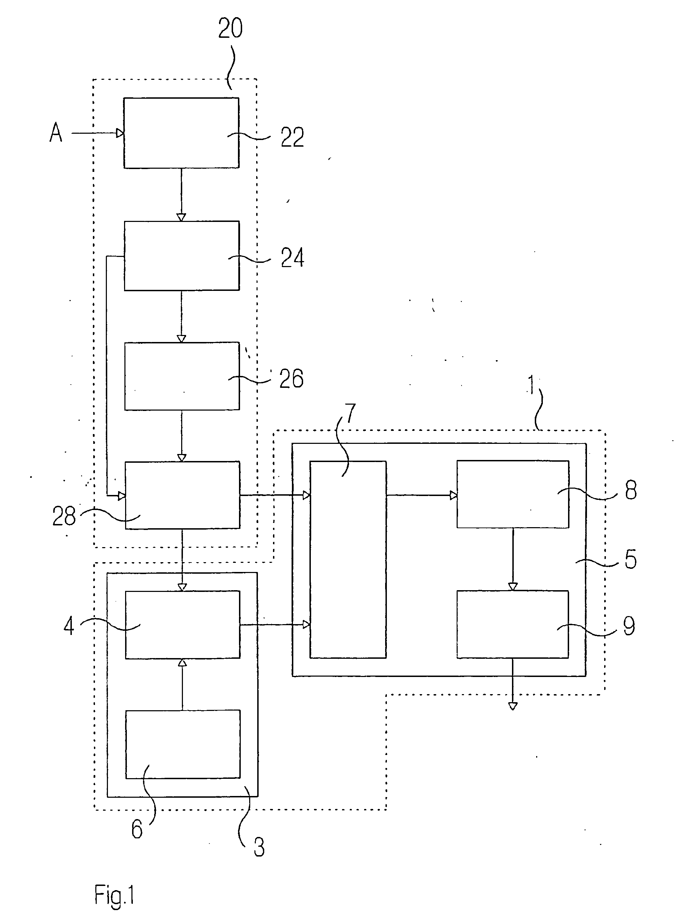

Fig. 1 zeigt ein Ausführungsbeispiel für die erfindungsgemäße Vorrichtung. Den Kern der Erfindungemäßen Vorrichtung bildet eine Klassifikationseinheit 1, die eine Transformationseinheit 3 sowie ein probabilistisches neuronales Netzwerk 5 umfasst, das mit der Transformationseinheit 3 zum Empfang von Koeffizienten (d.h. Werten), welche ein in die Transformationseinheit 3 eingehendes und ggf. aufbereitetes physiologisches Signal repräsentieren, verbunden ist. Der Transformationseinheit 3 ist eine Signalaufbereitungseinheit 20 vorgeschaltet, die im vorliegenden Ausführungsbeispiel einen Anti-Alias-Filter 22, einen Breitband-Analog-Digital-Wandler 24, im Folgenden kurz A/D-Wandler genannt, eine Detektionsstufe 26 zum Detektieren eines physiologischen Ereignisses, sowie eine kombinierte Justier/Normierstufe 28 umfasst, die von einem eingehenden physiologischen Eingangssignal A in dieser Reigenfolge durchlaufen werden, und die zum Ausgeben eines aufbereiteten physiologischen Signals mit der Transformationseinheit verbunden ist.

Im Folgenden wird die in der Signalaufbereitungseinheit 20 vorgenommene Signalaufbereitung des physiologischen Eingangssignals A, das im vorliegenden Ausführungsbeispiel ein intrakardiales Elektrogramm (IEGM) ist, wie es bspw. mittels eines Herzschrittmachers aufzunehmen ist, kurz erläutert. Jedoch sei darauf hingewiesen, dass die mit der vorliegenden Erfindung klassifizierbaren physiologischen Signale nicht auf intrakardiale Elektrogramme beschränkt sind.

Im Anti-Alias-Filter 22 erfolgt ein Filtern des IEGMs mittels eines Anti-Alias-Tiefpassfilters sowie ein geeignetes Verstärken und/oder Skalieren des IEGMs. Wie für Systeme abgetasteter Daten (sampled data systems) bekannt ist, unterdrückt der Filter Signalkomponenten, die bei Frequenzen oberhalb der halben Abtastrate auftreten können und von den nachfolgenden Signalverarbeitungsschritten aufgeprägt werden. Um die Genauigkeit und die Morphologie der Signalform des IEGMs zu erhalten, erfolgt darüber hinaus kein weiteres Filtern.

Das gefilterte IEGM wird von Anti-Alias-Filter 22 an den A/D-Wandler 24 weitergegeben, der ein konventioneller Analog-Digital-Wandler mit einer schrittweise linearen Beziehung zwischen dem Eingangssignal und dem Ausgangssignal ist. Die Abtastrate und die Auflösung des Ausgangssignals sind an die Anforderungen des Klassifizierungsprozesses angepasst. Im Allgemeinen liegen sie bei 1024 Hz oder darunter bzw. bei 8 Bit oder darüber. Abhängig von den Anforderungen können verschiedene A/D-Wandler-Architekturen zur Anwendung kommen, einschließlich des sog. "Ein-Bit-Designs" (One-Bit-Design). In speziellen Fällen, in denen Eingangssignale mit einem großen Dynamikbereich vorliegen, kann das Verwenden nichtlinearer (kompandierender, d.h. das Signal komprimierender und anschließend wieder expandierender) A/D-Wandlerstrukturen vorteilhaft sein. Das gewandelte IEGM wird vom A/D-Wandler 24 als Ausgangssignal an die Detektionsstufe 26 sowie an die Justier/Normierstufe 28 weitergeleitet.

In der Detektionsstufe 26 erfolgt die Detektion eines Ereignisses auf der Basis einer von Ereignis zu Ereignis ratenadaptiven Schwellenwertbetrachtung. Das Ergebnis der Detektion zeigt eine Aktivität der Signalform des Eingangssignals an. Wenn die Detektionsstufe 26 ein Ereignis detektiert, gibt sie ein Triggersignal (Auslösesignal) an die Justier/Normierstufe 28 aus, welches ein Justieren und/oder Normieren des physiologischen Signals auslöst.

Wenn die Justier/Normierstufe 28 ein Triggersignal von der Detektionsstufe 26 empfängt, so wird das zugrunde liegende IEGM in einem Ereignisfenster mit einer vorgegebenen Fensterweite, die im Allgemeinen 64 Abtastschritte beträgt, erfasst und im Fenster zentriert. Das Fenster ist an den erwarteten Ereignistyp angepasst. Es erfolgt außerdem ein Ermitteln des Zeitintervalls vom zuletzt detektierten Ereignis bis zum aktuellen Ereignis sowie eine Normierung der Signalform auf eine standardisierte Spitze-zu-Spitze-Amplitude anhand eines Normierungsfaktors, um ein normiertes Ereignissignal zu erhalten. Das Zeitintervall und den Normierungsfaktor gibt die Justier/Normierstufe 28 an das probabilistische neuronale Netzwerk 5 weiter, wohingegen sie das im Fenster zentrierte und normierte Ereignissignal an Transformationseinheit 3 weitergibt.

Die Transformationseinheit 3 nimmt eine Wavelet-Transformation des zentrierten und normierten Ereignissignals vor, die eine Anzahl das Signal repräsentierender Koeffizienten zum Ergebnis hat. Die Wavelet-Transformation ist ein wohlbekanntes Verfahren zum kompakten Darstellen beliebiger Signale. Dabei erfolgt die Transformation eines Signals mit Hilfe von Referenz-Wavelets und einem Berechnungsverfahren, das angibt, wie die Referenz-Wavelets mit dem Signal zu verrechnen sind. Die Details der Transformation können innerhalb der durch das Berechnungsumfeld gegebenen mathematischen Grenzen derart gewählt werden, dass sie sich für bestimmte Signalklassen sehr effektiv einsetzen lässt. Im vorliegenden Ausführungsbeispiel, das für den Einsatz in einer implantierbaren medizinischen Vorrichtung gedacht ist, ermöglicht die Wavelet-Transformation, ein Ereignisfenster mit einer Fensterweite von 64 Abtastschritten (64-Koeffizienten DWT) mit weniger als 16 Wavelet-Transformationskoeffizienten darzustellen und gleichzeitig genügend Information des Signals zu erhalten, um eine verlässliche Ereignisklassifikation im probabilistischen neuronalen Netzwerk 5 zu gewährleisten.

Zum Durchführen der Wavelet-Transformation umfasst die Transformationseinheit 3 einen Waveletspeicher 6, in dem die Referenz-Wavelets gespeichert sind, und eine Recheneinheit 4, die mit der Justier/Normierstufe 28 zum Empfang des im Fenster zentrierten und normierten Ereignissignals und mit dem Waveletspeicher 6 zum Empfang der Referenz-Wavelets verbunden ist. In der Recheneinheit 4 findet das Berechnen der Koeffizienten, d.h. die eigentliche Wavelet-Transformation statt.

Es existiert eine Anzahl Berechnungsverfahren, die zum Berechnen der Wavelet-Transformation geeignet sind. Ebenso existiert eine Vielzahl geeigneter Referenz-Wavelets. Für das Berechnen der Wavelet-Transformation in der Recheneinheit 4 kann der verwendete Satz Referenz-Wavelets bspw. im Hinblick auf die erzielbare Rechenleistung ausgewählt werden. Beim Auswählen des Berechnungsverfahrens und der Referenz-Wavelets ist jedoch vorzugsweise darauf zu achten, dass beim Berechnen der Wavelet-Transformation in der Recheneinheit 4 dasselbe Berechnungsverfahren und derselbe Satz Referenz-Wavelets zur Anwendung kommen, wie sie beim Berechnen der Vergleichskoeffizienten (siehe weiter unten) zur Anwendung gekommen sind.

Die Recheneinheit 4 gibt das Ergebnis der Wavelet-Transformation, also die Wavelet-Transformationskoeffizienten, als Satz von Koeffizienten an das probabilistische neuronale Netzwerk 5 (im Folgenden PNN abgekürzt) aus.

Das PNN 5 umfasst eine PNN-Struktur 8, eine Eingabeschicht 7 (input layer), sowie eine Ausgabeschicht oder Summationseinheit 9 (output layer). Die PNN-Struktur 8 weist eine Anzahl innerer Knoten auf und ist mit der Eingabeschicht 7 (input layer), die eine Anzahl Eingabeknoten aufweist, sowie mit der Summationseinheit 9, die eine Anzahl Ausgabeknoten aufweist, verbunden.

Die inneren Knoten der PNN-Struktur 8 enthalten jeweils einen bestimmten Koeffizientenvektor, der Vergleichskoeffizienten als ein Satz von Vergleichswerten, ein bestimmtes Vergleichszeitintervall sowie einen bestimmten Vergleichsnormierungsfaktor enthält und eine bestimmte Klasse von Ereignissen charakterisiert. Im vorliegenden Ausführungsbeispiel enthält die PNN-Struktur 8 für jede Klasse genau einen Knoten, es ist jedoch auch möglich einer Klasse mehrere innere Knoten mit jeweils leicht unterschiedlichen Koeffizientenvektoren zuzuordnen, so dass eine Klasse von einem Knoten-Cluster repräsentiert wird. Die Koeffizientenvektoren werden üblicherweise vorab aus einer Mehrzahl von Signalen mit der für die Ereignisklasse typischen Signalform extrahiert.

Aufgabe der Eingabeschicht 7 des PNN 5 ist es, von der Transformationseinheit 3 die Koeffizienten und von der Justier/Normierstufe 28 das Zeitintervall und den Normierungsfaktor des aktuellen IEGMs zu empfangen und gleichmäßig über die inneren Knoten der PNN-Struktur 8 zu verteilen.

In den inneren Knoten werden die jeweiligen Koeffizientenvektoren mit einem Signalvektor, der aus den von der Transformationseinheit 3 empfangenen Koeffizienten, sowie dem Zeitintervall und dem Normierungsfaktor des aktuellen IEGMs gebildet ist, verglichen, indem die Differenz aus Signalvektor und Koeffizientenvektor gebildet wird. Außerdem werden den ermittelten Vektordifferenzen Wahrscheinlichkeitswerte zugeordnet, wobei der Wahrscheinlichkeitswert umso größer ist, je geringer die Vektordifferenz ausfällt. Das Bestimmen der Wahrscheinlichkeitswerte kann eine gaußförmige Transferfunktion mit wählbarer Standardabweichung Sigma (die den Abstand der Wendepunkte der Kurve vom Kurvenzentrum angibt) einschließen. Die wählbare Standardabweichung ermöglicht es, die Grenzen, d.h. die maximal zulässige Abweichung von der jeweiligen Standardsignaiform einer Klasse festzulegen.

Die PNN-Struktur 8 ist zum weitergeben des Signalvektors sowie der für den Signalvektor ermittelten Wahrscheinlichkeitswerte mit der Summationseinheit 9 verbunden.

Die Summationseinheit 9 besitzt genau einen Ausgangsknoten für jede Ereignisklasse, für deren Erkennen die erfindungsgemäße Vorrichtung ausgestaltet ist. Der Ausgangsknoten empfängt den für die jeweilige Ereignisklasse ermittelten Wahrscheinlichkeitswert des Signalvektors. Falls einer Ereignisklasse mehrere innere Knoten zugeordnet sind, empfängt der entsprechende Ausgangsknoten alle Wahrscheinlichkeitswerte dieser inneren Knoten und berechnet den Mittelwert der entsprechenden Wahrscheinlichkeitswerte. In beiden Fällen stellt der Wahrscheinlichkeitswert eines Ausgangsknotens der Summationseinheit 9 die Wahrscheinlichkeit dafür dar, dass das das IEGM bzw. das auslösende Ereignis der durch den Ausgangsknoten repräsentierten Klasse angehört. Derjenigen Klasse, die in der Summationseinheit 9 den höchsten Wahrscheinlichkeitswert aufweist, wird das das IEGM auslösende Ereignis zugeordnet, sofern dieser Wahrscheinlichkeitswert eine Klassifizierungsschwelle überschreitet. Überschreitet er die Klassifizierungsschwelle nicht, so wird das Ereignis als unbekannt klassifiziert und ggf. dazu verwendet, eine Anpassung der PNN-Struktur auszulösen, die zum Erkennen einer neuen Ereignisklasse führt. Die Summationseinheit 9 gibt schließlich den Signalvektor und die Ereignisklasse, der er zugeordnet worden ist, als Ergebnis der Klassifikation aus.

Wie oben ausgeführt, enthält ein Knoten der PNN-Struktur 8 einen vorgegeben Koeffizientenvektor, welcher die von dem Knoten repräsentierte Ereignisklasse charakterisiert. Da diese Koeffizientenvektoren einer Ereignisklasse vorab aus einer repräsentativen Menge von Signalen, die auf die entsprechende Ereignisklasse zurückgehen, extrahiert worden sind, ist nicht zu erwarten, dass ein solcher Koeffizientenvektor ein einzelnes zu klassifizierenden Eingangssignal exakt wiedergibt. Entsprechend erfolgt das Klassifizieren, in der erfindungsgemäßen Vorrichtung anhand der Ähnlichkeit zwischen dem auf das Eingangssignal zurückgehenden Signalvektor, in dem die im Eingangssignal steckende Information kodiert ist, und den Koeffizientenvektoren der Knoten.

Die im Eingangssignal steckende Information wird nach dem Aufbereiten des Eingangsignals in der Signalaufbereitungseinheit 20 in der Transformationseinheit 3 mittels der Wavelet-Transformation kodiert. Die Wavelet-Transformation ist jedoch nicht invariant gegenüber einer zeitlichen Verschiebung des Eingangssignals im Signalfenster, d.h. das Ergebnis der Transformation ändert sich, wenn das Maximum der Amplitude um einen oder mehrere Abtastschritte im Signalfenster vor- oder rückverlegt wird. Infolgedessen können die Werte der von der Transformationseinheit ausgegebenen Koeffizienten, die für die Klassifikation von wesentlicher Bedeutung sind, fluktuieren. Das Ausmaß der Fluktuation hängt von der Genauigkeit der Zentrierung des Eingangssignals im Signalfenster ab. Obwohl die direkte Auswirkung dieser Fluktuation auf den Klassifizierungsvorgang in der Regel gering ist, können signifikante Klassifikationsfehler auftreten, wenn verrauschte Eingangssignale vorliegen.

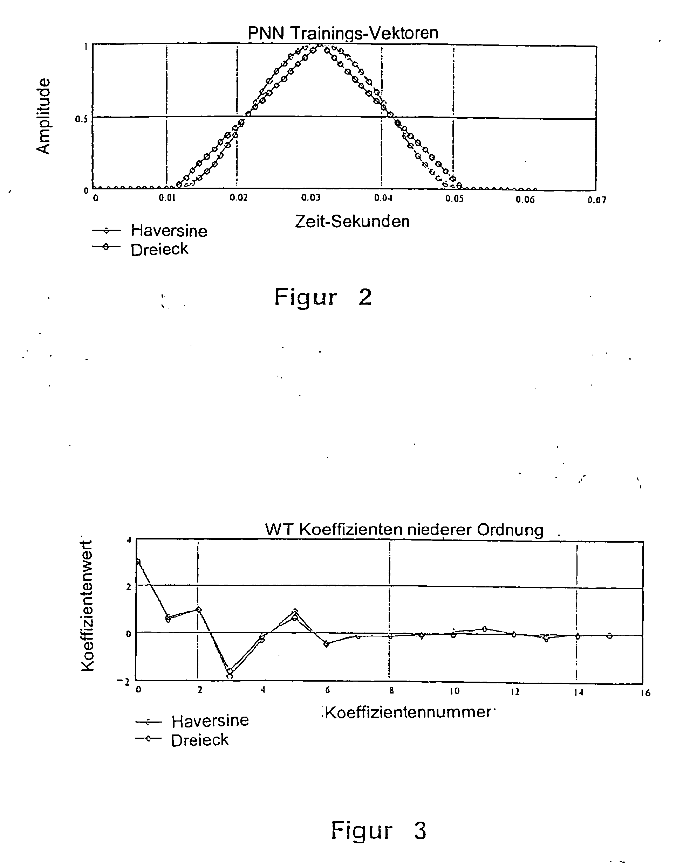

Nachfolgend wird eine mittels der erfindungsgemäßen Vorrichtung durchgeführte Klassifikation anhand der Figuren 2 bis 6, die Zwischenschritte im Ablauf der Klassifikation darstellen, anhand zweier verschiedner beispielhafter Test-Eingangssignale erläutert. Als Testsignale dienen einerseits ein sinusartiges sog. Haversine-Signal und andererseits ein Dreiecksignal. Dabei werden insbesondere die Konzepte zum Reduzieren des Einflusses von Faktoren, welche die Klassifikationsgenauigkeit verschlechtern, erläutert.

In Fig.2 sind das Haversine-Signal und das Dreiecksignal, die mit der erfindüngsgemäßen Vorrichtung unterschieden werden sollen, als Funktion der Zeit dargestellt. Für beide Signale beträgt die Signaldauer 40 ms, die Spitzenamplitude 1 V und die Abtastrate 1024 pro Sekunde. Die Grundform der beiden Signale ähnelt üblichen Signalformen intrakardialer Elektrogramme.

Figur 3 zeigt die Koeffizienten niedriger Ordnung, die für das Haversine-Signal bzw. das Dreiecksignal in der Transformationseinheit 3 mittels der Wavelet-Transformation gewonnen wurden. Dabei fand eine Daubechies-4-Transformation als Wavelet-Transformation Verwendung, die jeweils auf ein Haversine-Signal bzw. Dreiecksignal angewendet wurde, das zuvor in einem Signalfenster mit einer Weite von 64 Abtastschritten zentriert worden ist. Zwar sind die Ähnlichkeiten zwischen den jeweiligen Koeffizienten groß, jedoch sind hinreichende Unterscheide zwischen den Koeffizienten festzustellen. Andere Wavelet-Transformationen führen zu ähnlichen Ergebnissen wie die Daubechies-4-Transformation.

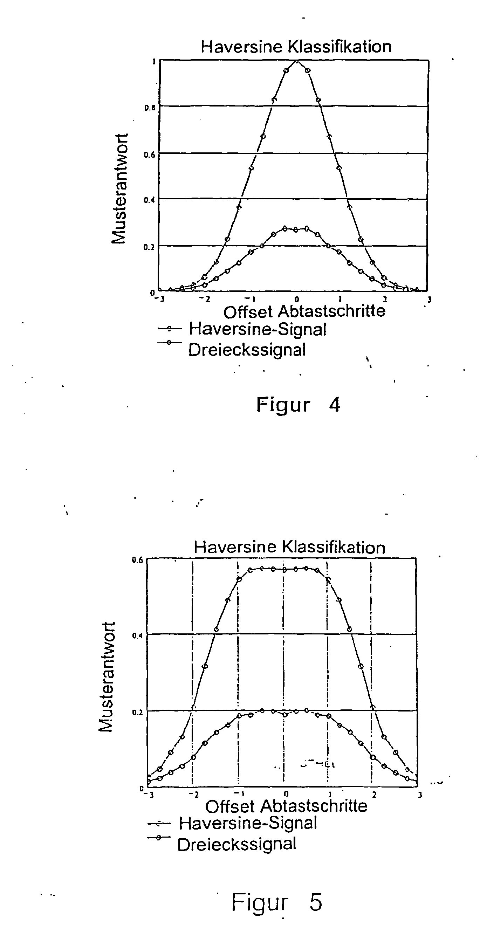

Fig. 4 zeigt sowohl für das Haversine-Signal als auch für das Dreiecksignal die Auswirkungen, die verschiedene Offsets (gemessen in Abtastschritten) beim Zentrieren des Signals im Signalfenster in einem Knoten der PNN-Struktur 8 mit einem ein Haversine-Signal repräsentierenden Koeffizientenvektor (d.h. der Knoten ist dazu ausgelegt, eine Ereignisklasse zu repräsentieren, die zu einem Haversine-Signal führt) hinterlassen.

Im Falle einer perfekten Zentrierung des Signals im Signalfenster, d.h. im Falle eines Offsets mit dem Wert Null, gibt der Knoten, der das Haversine-Signal repräsentiert, für das Harversine-Signal als Eingangssignal einen Ausgangswert 1 aus und für das Dreiecksignal als Eingangsignal einen Ausgangswert von etwa 0,25. Ist andererseits ein Offset um einen Abtastschritt in der Zentrierung des Haversine-Signals bzw. des Dreiecksignals im Signalfenster vorhanden (Offset +1 oder -1), so reduziert sich der Ausgangswert für das Haversine-Signal als Eingangssignal auf ca. 0,55 und der für das Dreiecksignal als Eingangsignal auf etwa 0,18.

Die Differenz zwischen den beiden in Fig. 4 dargestellten Ausgangswerten bildet die Grundlage der Ereignisklassifikation mit Hilfe des probabilistischen neuronalen Netzwerkes 5. Dabei gewährleistet eine große Differenz (wie beim Offset 0) eine geringe Anfälligkeit des Klassifikationsvorganges gegenüber Signalrauschen. Die Verringerung der Differenz aufgrund des Offsets von +1 oder -1 (gemessen in Abtastschritten) auf etwa die Hälfte des Wertes bei einem Offset von Null führt dazu, dass im Falle des Offsets von +1 bzw. -1 Klassifikationsfehler bereits bei Rauschpegeln auftreten, die halb so hoch sind, wie die Rauschpegel, bei denen Klassifikationsfehler bei einem Offset von Null auftreten. Dies kann bei manchen Anwendungen zu einer signifikanten Reduktion der Leistungsfähigkeit der Vorrichtung führen.

Wie erwähnt, ist es möglich, in der PNN-Struktur 8 einer Ereignisklasse mehrere innere Knoten mit jeweils leicht unterschiedlichen Koeffizientenvektoren zuzuordnen, so dass eine Ereignisklasse von einem Knoten-Cluster repräsentiert wird. Der Knoten-Cluster kann nun so ausgestaltet werden, dass einer Ereignisklasse zwei oder mehr Knoten zugeordnet sind, die jeweils einen Koeffizientenvektor enthalten, welcher einem (vorzugsweise geringen) Offset im zu erwartenden Eingangsignal entspricht. Die Koeffizientenvektoren werden üblicherweise vorab aus einer Mehrzahl von Signalen mit der für die Ereignisklasse typischen Signalform extrahiert, wobei die die Signale für verschieden Knoten eines Knoten-Clusters (leicht) gegeneinander zeitversetzt sind, d.h. einen Offset aufweisen. Die Größe des Zeitversatzes kann dabei im Hinblick auf die im Eingangssignal zu erwartenden Offsets, d.h. die zu erwartende Genauigkeit in der Zentrierung des Eingangssignals im Signalfenster, gewählt werden.

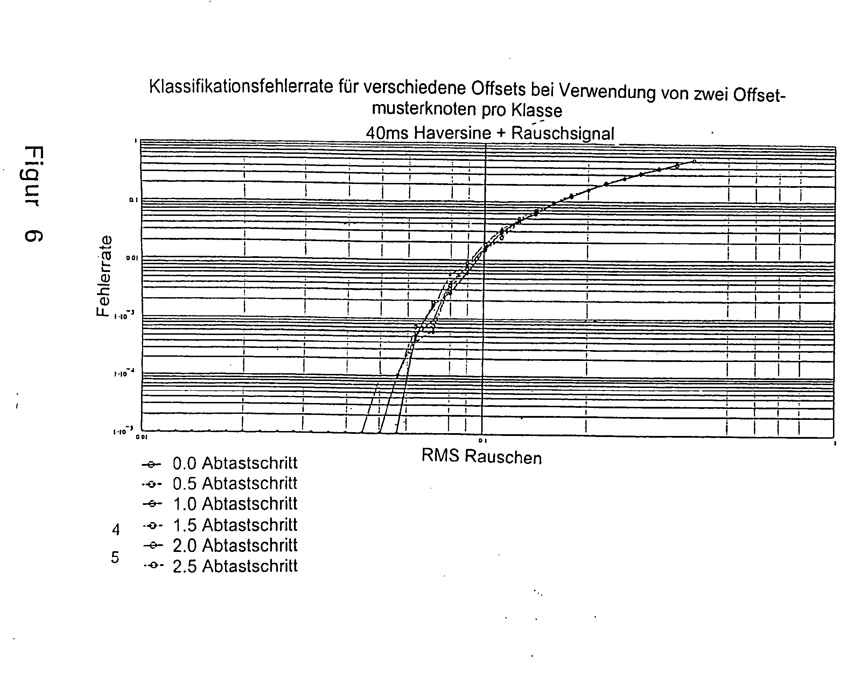

Im Ergebnis liefert ein derartiger Knoten-Cluster einen verbreiterten Bereich, in dem eine hohe Differenz zwischen seinem Ausgangswert bei einem Haversine-Signal als Eingangssignal und seinem Ausgangswert bei einem Dreiecksignal als Eingangsignal vorliegt (Fig. 5). Dadurch kann eine hohe Klassifikationsgenauigkeit auch bei Rauschpegeln, die sonst die Klassifikationsgenauigkeit signifikant beeinflussen würden, aufrechterhalten werden. Das Verhalten der Fehlerrate beim Klassifizieren in einem ein Haversine-Signal repräsentierenden Knoten-Cluster ist in Fig. 6 für verschiedene beim Zentrieren im Zeitfenster auftretende Offsets in Abhängigkeit vom Signalrauschen dargestellt.

## Patentansprüche

1. Vorrichtung zur Klassifikation physiologischer Ereignisse mit einem Signaleingang zum Eingeben eines ein physiologisches Ereignis darstellenden oder repräsentierenden physiologischen Signals und einer das physiologische Signal anhand seiner Signalform klassifizierende Klassifikationseinheit (1), wobei die Klassifikationseinheit (1) umfasst:
- eine Transformationseinheit (3), die derart zum Durchführen einer Transformation des physiologischen Signals ausgestaltet ist, dass sie als Ausgangssignal eine Anzahl von das physiologische Signal repräsentierenden und auf der Transformation beruhenden Werten ausgibt; und
- ein mit der Transformationseinheit (3) zum Empfang der Werte verbundenes probabilistisches neuronales Netzwerk (5), das eine Anzahl physiologische Ereignisse repräsentierender Ereignisklassen enthält, die wiederum jeweils durch einen Satz von Vergleichswerten repräsentiert sind, und das dazu ausgestaltet ist, anhand des Vergleichs der Werte mit den Vergleichswerten eine Zuordnung des von den Werten repräsentierten physiologischen Signals zu einer der Ereignisklassen vorzunehmen,
wobei die Transformationseinheit (3) zum Durchführen der Transformation anhand von Wavelets und einer die auszugebenden Werte unter Verwendung der Wavelets bestimmenden Transformationsregel ausgestaltet ist und wobei für mindestens eine Ereignisklasse zwei oder mehr Sätze von dieselbe Ereignisklasse repräsentierenden Vergleichswerten vorhanden sind,
**dadurch gekennzeichnet, dass** der Transformationseinheit (3) eine Justiereinheit (28) zum Zentrieren des physiologischen Signals in einem Zeitfenster vorgegebener Fensterweite und zum Ausgeben des zentrierten physiologischen Signals an die Transformationseinheit (3) vorgeschaltet ist und dass bei denjenigen Ereignisklassen, die zwei oder mehr Sätze von dieselbe Ereignisklasse repräsentierenden Vergleichswerten umfassen, die Sätze von Vergleichswerten unterschiedlichen, im Hinblick auf die zu erwartende Genauigkeit in der Zentrierung des Eingangssignals gewählten Offsets in der Zentrierung des zentrierten physiologischen Signals entsprechen.

2. Vorrichtung nach Anspruch 1, **dadurch gekennzeichnet, dass** die Vergleichswerte des probabillstischen neuronalen Netzwerks (5) auf einer Transformation beruhen, bei der die gleichen Wavelets und die gleiche Transformationsregel wie in der Transformationseinheit (3) Verwendung finden.

3. Vorrichtung nach Anspruch 1 oder 2, **dadurch gekennzeichnet, dass** das probabilistische neuronale Netzwerk (5) umfasst:
- mindestens eine Ermittlungseinheit (8) zum Bestimmen von Zugehörigkeitswahrscheinlichkeiten des physiologischen Signals zu den Ereignisklassen auf der Basis des Vergleichs der Werte mit den Vergleichswerten der jeweiligen Ereignisklasse und zum Ausgeben der ermittelten Zugehörigkeitswahrscheinlichkeiten; und
- eine mit der Ermittlungseinheit zum Empfang der Zugehörigkeitswahrscheinlichkeiten verbundene Selektionseinheit (9), die zum Extrahieren der höchsten Zugehörigkeitswahrscheinlichkeit aus den Zugehörigkeitswahrscheinlichkeiten sowie zum Zuordnen des physiologischen Signals zu der Ereignisklasse mit der höchsten Zugehörigkeitswahrscheinlichkeit ausgebildet ist.

4. Vorrichtung nach Anspruch 1 und Anspruch 3, **dadurch gekennzeichnet, dass** die Ermittlungseinheit (8) zum Bestimmen mehrerer Zugehörigkeitswahrscheinlichkeiten für jede Ereignisklasse ausgebildet ist, die zwei oder mehr Sätze von dieselbe Ereignisklasse repräsentierenden Vergleichswerten aufweist, und die Selektionseinheit (9) derart ausgebildet ist, dass sie für diejenigen Ereignisklassen, die zwei oder mehr Sätze von dieselbe Ereignisklasse repräsentierenden Vergleichswerten aufweisen, Mittelwerte der entsprechenden Zugehörigkeitswahrscheinlichkeiten bildet und beim Extrahieren der höchsten Zugehörigkeitswahrscheinlichkeit die Mittelwerte statt der Einzelwerte heranzieht.

5. lmplantierbares medizinisches Gerät, **dadurch gekennzeichnet, dass** es mit einer Vorrichtung zur Klassifikation physiologischer Ereignisse nach einem der Ansprüche 1 bis 4 ausgestattet ist.

6. lmplantierbares medizinisches Gerät nach Anspruch 5, **gekennzeichnet durch** seine Ausgestaltung als Herzschrittmacher oder Defibrillator.

## Claims

1. A device for classifying physiological events comprising a signal input for the input of a physiological signal that depicts or represents a physiological event, and comprising a classification unit (1) that classifies the physiological signal on the basis of its waveform, wherein the classification unit (1) includes:
- a transformation unit (3) designed to transform the physiological signal such that it outputs, as the output signal, a number of values that represent the physiological signal and are based on the transformation; and
- a probabilistic neural network (5) that is connected to the transformation unit (3) to receive the values, and that contains a number of event classes that represent physiological events, each event class being represented by a set of comparison values, and that is designed to assign the physiological signal represented by the values to one of the event classes on the basis of the comparison of the values with the comparison values,
wherein the transformation unit (3) is designed to carry out the transformation on the basis of wavelets and a transformation rule that determines the values to be output using the wavelets, and wherein two or more sets of comparison values, which represent the same event class, are provided for at least one event class,
**characterized in that** an adjustment unit (28) for centering the physiological signal in a time window having a predefined with, and for outputting the centered physiological signal is installed upstream of the transformation unit (3), and **in that**, for those event classes that include two or more sets of comparison values representing the same event class, the sets correspond to offsets in the centering of the centered physiological signal that differ from comparison values and are selected in terms of the expected accuracy in the centering of the input signal.

2. The apparatus according to claim 1, **characterized in that** the comparison values of the probabilistic neural network (5) are based on a transformation in which the same wavelets and the same transformation rule are used as in the transformation unit (3).

3. The apparatus according to claim 1 or 2, **characterized in that** the probabilistic neural network (5) includes:
- at least one determination unit (8) for determining probabilities that the physiological signal belongs to the event classes on the basis of the comparison of the values with the comparison values of the particular event class, and for outputting the association probabilities that were determined; and
- a selection unit (9) connected to the determination unit for receiving the association probabilities, which is designed to extract the greatest association probability from the association probabilities, and to assign the physiological signal to the event class having the greatest association probability.

4. The apparatus according to claim 1 and claim 3, **characterized in that** the determination unit (8) is designed to determine a plurality of association probabilities for each event class that contains two or more sets of comparison values that represent the same event class, and the selection unit (9) is designed such that it averages the corresponding association probabilities for the event classes that contain two or more sets of comparison values that represent the same event class, and uses the means instead of the individual values when extracting the highest association probability.

5. An implantable medical device, **characterized in that** it is equipped with an apparatus for classifying physiological events according to one of the claims 1 through 4.

6. The implantable medical device according to claim 5, **characterized by** its embodiment as a cardiac pacemaker or defibrillator.

## Revendications

1. Dispositif pour la classification d'évènements physiologiques, comprenant une entrée de signal pour l'entrée d'un signal physiologique décrivant ou représentant un évènement physiologique, et comprenant une unité de classification (1) classifiant le signal physiologique sur la base de sa forme d'onde, dans lequel l'unité de classification (1) comprend:
- une unité de transformation (3) conçue pour transformer le signal physiologique de manière à émettre, en tant que signal de sortie, un nombre de valeurs représentant le signal physiologique et étant basées sur la transformation; et
- un réseau neural probabiliste (5), relié à l'unité de transformation (3), pour recevoir les valeurs, et contenant un nombre de classes d'évènements représentant des évènements physiologiques, chaque évènement étant représenté par une série de valeurs de comparaison, et conçu pour attribuer le signal physiologique représenté par les valeurs à l'une des classes de valeurs, sur la base de la comparaison des valeurs avec les valeurs de comparaison,
dans lequel l'unité de transformation (3) est conçue pour exécuter la transformation sur la base d'ondelettes et d'une règle de transformation déterminant les valeurs à émettre à l'aide des ondelettes, et dans lequel deux ou plusieurs séries de valeurs de comparaison, représentant la même classe d'évènement, sont fournies pour au moins une classe d'évènement, **caractérisé en ce que**
une unité d'ajustement (28) pour le centrage du signal physiologique dans une fenêtre temporelle possédant une largeur prédéterminée, et pour la sortie du signal physiologique centré, tout en étant installée en amont de l'unité de transformation (3), et **en ce que**, pour les classes d'évènements comprenant deux ou plusieurs séries de valeurs de comparaison représentant la même classe d'évènement, les séries correspondent à des décalages dans le centrage du signal physiologique centré, différentes des valeurs de comparaison et sélectionnées en termes d'exactitude attendue dans le centrage du signal d'entrée.

2. Dispositif selon la revendication 1, **caractérisé en ce que** les valeurs de comparaison du réseau neural probabiliste (5) sont basées sur une transformation dans laquelle les mêmes ondelettes et la même règle de transformation sont utilisées comme dans l'unité de transformation (3).

3. Dispositif selon la revendication 1 ou 2, **caractérisé en ce que** le réseau neural probabiliste (5) comprend :
- au moins une unité de détermination (8) pour déterminer les probabilités selon lesquelles le signal physiologique appartient aux classes d'évènements, sur la base de la comparaison entre les valeurs et les valeurs de comparaison de la classe d'évènement particulière, et pour émettre les probabilités d'association déterminées ;
- une unité de sélection (9) reliée à l'unité de détermination pour recevoir les probabilités d'association, conçue pour extraire la plus grande probabilité d'association à partir des probabilités d'association, et pour attribuer le signal physiologique à la classe d'évènement présentant la plus grande probabilité d'association.

4. Dispositif selon la revendication 1 et la revendication 3, **caractérisé en ce que** l'unité de détermination (8) est conçue pour déterminer une pluralité de probabilités d'association pour chaque classe d'évènement contenant deux ou plusieurs séries de valeurs de comparaison représentant la même classe d'évènement, et l'unité de sélection (9) est conçue de manière à faire la moyenne des probabilités d'association pour les classes d'évènements contenant deux ou plusieurs séries de valeurs de comparaison représentant la même classe d'évènement, et à utiliser la moyenne à la place des valeurs individuelles lors de l'extraction de la plus grande probabilité d'association.

5. Dispositif médical implantable, **caractérisé en ce que** il est équipé d'un dispositif pour la classification d'évènements physiologiques selon l'une des revendications 1 à 4.

6. Dispositif médical implantable selon la revendication 5, **caractérisé par** sa forme de réalisation en tant que stimulateur cardiaque ou défibrillateur.
